# FASCICULE DE BREVET EUROPEEN

(11) **EP 2 134 316 B1**
(45) Date de publication et mention de la délivrance du brevet: **19.11.2014**
(21) Numéro de dépôt: 08761855.9
(22) Date de dépôt: 08.02.2008
(51) Int. Cl.: A61K 8/27, A61K 8/97, A61Q 5/00, A61Q 7/00, A61Q 19/00, A61K 36/00, A61K 36/76, A61K 36/896, A61K 36/752, A61K 36/75

(54) **NOUVELLES COMPOSITIONS COSMETIQUES ET/ OU PHARMACEUTIQUES ET LEURS APPLICATIONS**
NEUARTIGE KOSMETISCHE UND/ODER PHARMAZEUTISCHE ZUSAMMENSETZUNGEN UND DEREN VERWENDUNGEN
NOVEL COSMETIC AND/OR PHARMACEUTICAL COMPOSITIONS AND APPLICATIONS THEREOF

(30) Priorité: 13.02.2007 FR 0701011
(43) Date de publication de la demande: 23.12.2009
(60) Demande divisionnaire: 12186394.8
(73) Titulaire: Legacy Healthcare Ltd, Malta, VLT 1165 (MT)
(72) Inventeur: ULMANN, André, F-75005 Paris (FR); STURER, Jean-Frédéric, F-75005 Paris (FR)
(74) Mandataire: Hirsch & Associés
(86) Numéro de dépôt international: PCT/FR2008/000152
(87) Numéro de publication internationale: WO 2008/113912

(56) Documents cités:
- WO-A-2005/120452
- CH-A5- 682 217
- DE-A1- 2 844 614
- DE-A1- 19 533 777
- DE-A1-102004 011 968
- DE-A1-102005 010 142
- DE-U-202004 012 348
- FR-A1- 2 706 771
- FR-A1- 2 877 219
- FR-A1- 2 877 576
- US-A1- 2003 077 336
- DATABASE WPI Week 200708 Thomson Scientific, London, GB; AN 2007-076113 XP002451092 & JP 2006 104098 A (ICHIMARU PHARCOS INC) 20 avril 2006 (2006-04-20)
- DATABASE WPI Week 200377 Thomson Scientific, London, GB; AN 2003-819640 XP002451093 & JP 2003 201229 A (SHISEIDO CO LTD) 18 juillet 2003 (2003-07-18)
- DATABASE WPI Week 200706 Thomson Scientific, London, GB; AN 2007-050778 XP002451094 & JP 2006 342120 A (WISE CONNECTION KK) 21 décembre 2006 (2006-12-21)
- DATABASE WPI Week 200019 Thomson Scientific, London, GB; AN 2000-217905 XP002451095 & JP 2000 044439 A (SHISEIDO CO LTD) 15 février 2000 (2000-02-15)
- DATABASE WPI Week 200066 Thomson Scientific, London, GB; AN 2000-675233 XP002451096 & JP 2000 247830 A (NAGASE SANGYO KK) 12 septembre 2000 (2000-09-12)
- TKDL: 'Tila-e- Dohn-ul- Utraj' PAN: JA3/187C 01 Janvier 1874, XP002698397

## Description

L'invention concerne de nouvelles compositions, ainsi que l'utilisation de ces compositions en cosmétique et/ou leur application à titre de médicaments.

Un certain nombre de compositions cosmétiques et/ou pharmaceutiques renfermant de l'oignon frais, du sel iodé, de l'acide citrique et du jus de citron ont été décrites dans la littérature. Ces compositions peuvent être utilisées notamment en cosmétique pour favoriser la repousse des cheveux ou dans des indications thérapeutiques pour favoriser l'angiogènèse, pour stimuler la synthèse du collagène, pour stimuler la prolifération et l'activation des mastocytes, voire dans le traitement de certains cancers. Parmi les références de la littérature, on peut citer par exemple les demandes de brevet français N° 2 875 403, 2 877 219, 2 877 224, 2 877 576, 2 877 408 ou la demande de brevet Suisse N° 682 217 A.

La demanderesse a étudié de nouvelles compositions pouvant être utilisées en cosmétique ou en thérapeutique humaine qui présentent des activités très supérieures, une grande stabilité et qui ne présentent pas les inconvénients des compositions décrites dans la littérature.

L'invention a ainsi pour objet de nouvelles compositions, caractérisées en ce qu'elles comprennent à titre de principes actifs un mélange consistant en un extrait *d'Allium species,* un extrait de *Citrus species,* un extrait de *Paullinia species* et un extrait de *Theobroma species.*

Parmi ces compositions, on retient plus particulièrement les compositions telles que définies ci-dessus, caractérisées en ce qu'elles consistent en un extrait hydroalcoolique *d'Allium species,* un extrait hydroalcoolique de *Citrus species,* un extrait hydroalcoolique, atomisé ou non, de *Paullinia species* et un extrait hydroalcoolique, atomisé ou non, de *Theobroma species.*

Parmi les compositions de l'invention, on retient tout particulièrement les compositions telles que définies ci-dessus, renfermant: de 65 % à 93 % d'un extrait hydroalcoolique *d'Allium species,* de 5 % à 33 % d'un extrait hydroalcoolique de *Citrus species,* de 0,25 % à 2,5 % d'un extrait hydroalcoolique, atomisé ou non, de *Paullinia species* et de 0,25 % à 2,5 % d'un extrait hydroalcoolique, atomisé ou non, de *Theobroma species* et notamment celles renfermant de 65 % à 93 % d'un extrait hydroalcoolique *d'Allium cepa,* de 5 % à 33 % d'un extrait hydroalcoolique de *Citrus lemon,* de 0,25 % à 2,5 % d'un extrait hydroalcoolique, atomisé ou non, de *Paullinia species,* et de 0,25 % à 2,5 % d'un extrait hydroalcoolique, atomisé ou non, de *Theobroma species.*

Par extrait *d'Allium species* ou extrait hydroalcoolique *d'Allium species,* on désigne notamment les extraits hydroalcooliques ainsi que les extraits natifs obtenus à partir de toutes les espèces du genre *Allium* (famille des Liliaceae) et notamment *d'Allium cepa.*

Par extrait de *Citrus species* ou extrait hydroalcoolique de *Citrus species,* on désigne notamment les extraits hydroalcooliques ainsi que les extraits natifs obtenus à partir de toutes les espèces du genre *Citrus* (famille des Rutacae) et notamment de *Citrus lemon.*

Par extrait, atomisé ou non, de *Paullinia species,* ou extrait hydroalcoolique, atomisé ou non, de *Paullinia species,* on désigne notamment les extraits hydroalcooliques ainsi que les extraits natifs obtenus à partir de toutes les espèces du genre *Paullinia* (famille des Sapindaceae) et notamment de *Paullinia cupana.*

Par extrait, atomisé ou non, de *Theobroma species* ou extrait hydroalcoolique, atomisé ou non, de *Theobroma species,* on désigne notamment les extraits hydroalcooliques ainsi que les extraits natifs obtenus à partir de toutes les espèces du genre *Theobroma* (famille des Malvaceae) et notamment de *Theobroma cacao.*

L'invention a également pour objet un procédé de préparation des compositions telles que définies ci-dessus, caractérisé en ce que l'on prépare une liqueur mère renfermant les extraits *d'Allium species* et de *Citrus species,* puis mélange de cette liqueur mère avec un extrait de *Paullinia species* et un extrait de *Theobroma species,* pour obtenir la composition recherchée.

Selon des conditions préférentielles de mise en oeuvre du procédé, ce procédé est caractérisé en ce que la liqueur mère est préparée par épluchage, nettoyage, broyage et macération des *Alliums* et des *Citrus* pendant plusieurs heures dans l'alcool, filtration puis ajustement de l'extrait alcoolique et mélange avec les autres ingrédients.

Dans la mise en oeuvre du procédé de préparation des compositions objet de la présente demande, on peut avantageusement préparer la liqueur mère au départ des éléments constitutifs, par épluchage, nettoyage, broyage des *Alliums* et des *Citrus,* mise en présence d'alcool à 96°, puis macération à froid pendant 48 heures, filtration de la liqueur mère sur des filtres 0,22 micron, pour recueillir un extrait hydro-alcoolique renfermant un faible taux de particules, puis ajuste l'extrait obtenu à 30° alcooliques. On ajoute alors à cette liqueur mère, par simple mélange, les extraits, atomisés ou non, de *Paullinia species* et de *Theobroma species.*

Les compositions telles que définies ci-dessus présentent d'intéressantes propriétés qui les rendent aptes à être utilisées en cosmétique.

Dans les applications cosmétiques, ces compositions peuvent notamment être destinées à réguler et/ou stabiliser toutes atteintes de la peau, du cuir chevelu ou des phanères, et notamment à ralentir la chute des cheveux, à stimuler leur croissance, à augmenter leur densité, à éliminer les pellicules, à palier aux phénomènes de vieillissement du cuir chevelu, et à palier à toutes atteintes aux ongles.

Dans le cas d'une application topique, ces compositions peuvent ainsi être appliquées directement sur la peau ou le cuir chevelu à raison d'une à trois applications par jour sous forme de solutions ou de lotions concentrées par exemple de 5 à 30 % et de préférence autour de 20 %.

Dans le cas d'une utilisation en cosmétique, les compositions peuvent aussi être administrées sous forme de compléments alimentaires sous la forme de granulés, de gélules souples ou sous forme de boissons ; selon la forme finale recherchée, au lieu d'utiliser le produit sous forme de solution hydroalcoolique, on pourra avantageusement utiliser le produit sous une forme sèche obtenue au départ de la précédente, par exemple par atomisation, lyophilisation, concentration ou extraction secondaire par CO2 super critique de ladite solution hydroalcoolique.

Les compositions telles que définies ci-dessus présentent également de très intéressantes propriétés pharmacologiques.

De ce fait, elles peuvent être administrées chez l'homme ou l'animal, à titre de médicaments, notamment à titre thérapeutique ou prophylactique.

Elles peuvent en particulier être utilisées pour traiter les dermatoses et plus particulièrement les dermatoses dues à des infections bactériennes et virales cutanées diverses, les vieillissements cutanés, le blanchiment et le whitening au niveau de la peau, le vitiligo, les acrodermatites, le vieillissement cutané photo induit (actinique) ainsi que les atteintes solaires diverses (photodermatoses), les acnés de toutes origines (vulgaris, inflammatoires ou papulo pustule), l'acné rosacée, les lupus érythémateux, les peaux sensibles, les piqûres d'insectes, les peaux grasses ou sèches, les troubles de la séborrhée, les alopécies de toutes origines, la chute des cheveux, le pityriasis capitis, la pelade, le cuir chevelu sensible, les cheveux gras, le psoriasis et le para psoriasis du cuir chevelu, les dyshidroses, les verrues et les cors, la gale, la perlèche et les oedèmes, ou toutes maladies pouvant affecter les ongles.

En thérapeutique, ces compositions ont également un effet sur la microvascularisation et un effet sur la cicatrisation des plaies, des affections de toutes origines, des brûlures, la couperose, la maladie de Behcet, la porphyrie cutanée et les angiomes ainsi que les métastases cancéreuses.

Elles peuvent encore être utilisées en thérapeutique pour traiter les troubles de la kératinisation, ainsi que les lésions kératosiques, les hyperkératoses dont la kératodermie, les dyskératoses, la maladie de Darier, les parakératoses, les ichtyoses, les eczémas, les nevrodermites, les érythrodermites, les lichenifications, les dermatoses squameuses, les prurigos, les chéloïdes, les pustuloses.

Elles peuvent enfin être utilisées en thérapeutique pour traiter les troubles circulatoires et vasomoteurs et notamment les signes fonctionnels pendant les poussées hémorroïdaires, les affections thrombotiques, les symptômes avec insuffisances veino-lymphatiques, les jambes lourdes, la paresthésie.

Elles peuvent être administrées telles quelles ou en mélanges avec un ou plusieurs autres composés sous la forme d'une composition pharmaceutique qui renferme à titre de composé actif une dose efficace ainsi que des excipients et/ou additifs courants et pharmaceutiquement inertes.

Ces compositions pharmaceutiques peuvent être administrées par voie buccale, par voie entérale ou parentérale ou par voie locale en application topique sur la peau et les muqueuses ou par injection par exemple par voie sous cutanée.

Les médicaments peuvent être administrés oralement, par exemple sous forme de comprimés, de comprimés enrobés, de pelliculés, de granules, de gélules et capsules molles, de solutions, de sirops, d'émulsion, de suspension ou de mélange d'aérosol. Comme indiqué plus haut, en fonction de la forme recherchée, on pourra avantageusement utiliser le produit sous forme de solution hydroalcoolique ou sous forme sèche.

L'administration peut également être effectuée par voie rectale, par exemple sous forme de suppositoires ou par voie parentérale, par exemple sous forme de solutions injectables ou d'infusions, de microcapsules ou d'implants, par voie percutanée, par exemple sous la forme de pommade, de solutions, de pigments ou de colorants, par voie transdermique (patches) ou par d'autres voies telles que sous la forme d'aérosol ou de spray nasal.

Ces compositions pharmaceutiques sont préparées selon des méthodes usuelles, des excipients organiques ou inorganiques, pharmaceutiquement inertes étant ajoutés aux compositions obtenues selon l'invention.

Ces compositions peuvent donc être solides ou liquides et se présenter sous toutes les formes pharmaceutiques couramment utilisées en médecine humaine comme, par exemple, les comprimés simples ou dragéifiés, les pilules, les tablettes, les gélules, les gouttes, les granulés, les préparations injectables, les pommades, les crèmes ou les gels ; elles sont préparées selon les méthodes usuelles.

Pour la production de comprimés, simples ou dragéifiés, de comprimés enrobés et de capsule en gélatine dure, il est possible d'utiliser par exemple, du lactose, de l'amidon de maïs ou ses dérivés, du talc, de l'acide stéarique ou ses sels.

Les supports convenables pour des capsules en gélatine molle ou pour les suppositoires sont par exemple les graisses, les cires les polyols semi-solides ou liquides, les huiles naturelles ou modifiées etc. Les véhicules appropriés pour la préparation de solutions, par exemple les solutions injectables, les émulsions ou les sirops sont par exemple l'eau, les alcools, le glycérol, les polyols, le sucrose, les sucres invertis, le glucose, les huiles végétales, etc. Les supports convenables pour les microcapsules ou les implants sont par exemple les copolymères d'acide glyoxilique et d'acide lactique.

Les principes actifs tels que définis ci-dessus peuvent y être incorporé à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

En plus des principes actifs tels que définis ci-dessus et des excipients, les compositions pharmaceutiques peuvent contenir des additifs tels que par exemple des diluants, des désintégrants, des liants, des lubrifiants, des agents mouillant, des stabilisants, des émulsifiants, des préservateurs, des agents sucrant, des colorants des agents de flaveurs ou des aromatisants, des épaississants, des agents tampons, et aussi des solvants ou des solubilisants ou des agents pour obtenir un effet retard et également des sels pour modifier la pression osmotique, des agents d'enrobage ou des antioxydants.

La présente invention s'étend ainsi aux compositions pharmaceutiques contenant à titre de principe actif l'un au moins des médicaments tels que définis ci-dessus.

Lorsqu'on utilise les compositions obtenues selon l'invention, les doses peuvent varier à l'intérieur de limites assez larges et doivent être fixées en fonction de la personne à traiter et de l'affection en cause. Les compositions pharmaceutiques renferment normalement de 0,2 à 500 mg, et de préférence de 1 à 200 mg de principes actifs tels que définis ci-dessus, sous forme d'extrait sec. Dans le cas d'une administration par voie orale, la dose quotidienne varie en général de 0,05 à 10 mg/kg et de préférence de 0,1 à 8 mg/kg, en particulier de 0,1 à 6 mg/kg. Par exemple pour un adulte on pourra envisager une dose quotidienne variant de 5 à 500 mg.

Dans le cas d'une administration par voie intraveineuse, la dose quotidienne varie approximativement de 0,05 à 6 mg/kg et de préférence de 0,1 à 5 mg/kg.

La dose quotidienne peut être divisée, en particulier dans le cas de l'administration de grande quantité de principe actif.

Il va être donné maintenant dans ce qui suit, à titre non limitatif, des exemples de mise en oeuvre de l'invention. Compte tenu de la nature des produits, il va sans dire que, lorsque l'on aura à utiliser les produits sous une forme liquide ou solide, on prendra le soin d'y ajouter un arôme ou un parfum.

### EXEMPLES :

### Exemple 1 :

On a préparé une composition renfermant :
- un extrait hydroalcoolique *d'Allium cepa : 87, 04 %*
- un extrait hydroalcoolique de *Citrus lemon : 11,96 %*
- un extrait hydroalcoolique atomisé de *Paullinia cupana : 0,50 %*
- un extrait hydroalcoolique atomisé de *Theobroma cacao : 0,50 %*

On prépare l'extrait hydroalcoolique *d'Allium cepa* par épluchage, nettoyage, broyage des *Alliums* et macération pendant 48 heures à froid dans de l'alcool 96°. On filtre sur filtre 0,22 micron, recueille le filtrat alcoolique et ajuste à 30° alcoolique.

On prépare l'extrait hydroalcoolique de *Citrus lemon* par épluchage, nettoyage, broyage des *Citrus* et macération pendant 48 heures à froid dans de l'alcool 96°. On filtre sur filtre 0,22 micron, recueille le filtrat alcoolique et ajuste à 30° alcoolique.

On prépare par simple mélange une liqueur mère renfermant les extraits hydroalcooliques *d'Alliums* et de *Citrus.* A cette liqueur mère on ajoute les extraits hydroalcooliques atomisés de *Paullinia cupana* et de *Theobroma cacao.* On recueille l'extrait ainsi obtenu.

### Exemple 3 :

On a préparé un complément alimentaire renfermant :
- un extrait sec *d'Allium cepa : 87,04* %
- un extrait sec de *Citrus lemon : 11,96 %*
- un extrait sec de *Paullinia cupana : 0,50 %*
- un extrait sec de *Theobroma cacao : 0,50* %

On lyophilise la composition obtenue à l'exemple 1 après y avoir ajouté un arôme pour obtenir une poudre que l'on peut utiliser directement à titre de complément alimentaire.

### Exemple 4 :

On a préparé une lotion cosmétique destinée à traiter le cuir chevelu :
- composition obtenue à l'exemple 1 21 ml
- excipient qsp (dont parfum/arôme) 100 ml

La lotion est préparée par simple mélange d'une composition obtenue à l'exemple 1 et d'un excipient.

### Exemple 6 :

On a préparé des comprimés répondant à la formule :
- composition obtenue à l'exemple 3 100 mg
- excipient qsp (dont parfum/arôme) 300 mg

### (Détail de l'excipient : lactose, talc, amidon, stéarate de magnésium)

### Exemple 7 :

On a préparé une pommade destinée à être utilisée à titre de médicaments :
- composition obtenue à l'exemple 3 5 ml
- excipient qsp (dont parfum/arôme) 100 g

### 1 : Etude de l'efficacité anti-chute par la méthode du photo trichogramme d'un traitement capillaire utilisé dans les conditions normales d'emploi pendant 12 semaines consécutives.

Vingt volontaires, de sexe masculin, âgés de 35 à 55 ans et présentant une calvitie légère à modérée, correspondant aux stades II à IV de la classification de Hamilton ont été traités par la composition de l'exemple 4 (Classification de Hamilton : stade I : creusement des golfes temporaux, stade II : creusement des golfes temporaux et éclaircissement de la zone du tourbillon, stade III : confluence de la zone des golfes et du tourbillon, stade IV : calvitie sauf sur la zone temporo-occipitale, stade V : couronne occipitale).

Le protocole a été le suivant :
J1 : repérage du site temporal, section des cheveux à l'émergence du cuir chevelu, macrophotographie de la zone étudiée,
J1 - J2 : pas d'application du produit, pas de shampoing,
J3 : nouvelle macrophotographie de la même zone qu'à J1 et début du traitement :
J3 à J42 : application du produit sur le cuir chevelu 2 fois par jour (2 à 3 pulvérisations à chaque fois),
J42 : évaluation de la tolérance capillaire et cutanée, repérage du site temporal, section des cheveux à l'émergence du cuir chevelu, macrophotographie de la zone étudiée,
J42-J43 : pas d'application du produit et pas de shampoing,
J44 : nouvelle macrophotographie de la même zone qu'à J1,
J44-J84 : application du produit étudié sur le cuir chevelu 2 fois par jour (2 à 3 pulvérisations à chaque fois), au site section des cheveux à l'émergence du cuir chevelu,
J84 : évaluation de la tolérance capillaire et cutanée, repérage du site temporal de la zone étudiée, section des cheveux à l'émergence du cuir chevelu, macrophotographie de la zone étudiée,
J84-J85 : pas d'application du produit et pas de shampoing,
J86 : nouvelle macrophotographie de la même zone qu'à J1.

L'étude a montré :
- une importante efficacité antichute après 6 semaines de traitement : augmentation statistiquement significative du ratio A/T (rapport anagène/télogéne) de + 44,91 %
- une importante efficacité anti-chute après 12 semaines de traitement : augmentation statistiquement significative du ratio A/T (rapport anagène/télogéne) de + 46,78 %
- une bonne tolérance clinique : aucune réaction cutanée n'a été constatée lors de l'examen dermatologique
- une bonne tolérance subjective clinique : un volontaire a toutefois décrit des manifestations d'inconfort cutané.

### 2) Evaluation de l'activité angiogénique des composés de l'exemple 1

Le produit de l'invention a été testé sur des plaques « Angiokits ». selon des tests bien connus, on contrôle le développement de cellules endothéliales en co-culture avec des fibroblastes et observe la formation de structures capillaires (tubules). Le produit testé à différentes concentrations est introduit sur chaque plaque dans certains puits. Certains puits reçoivent en outre un activateur (VEGF) alors que d'autres reçoivent un inhibiteur (suramine). Certains puits servent de témoins.

On change le milieu nutritif aux jours 3, 4, 7, 10 et 12 et arrête l'essai au jour 15.

Les résultats observés au jour 15, montrent que le produit présente une forte activité angiogénique.

## Revendications

1. Compositions comprenant à titre de principes actifs un mélange consistant en un extrait *d'Allium species,* un extrait de *Citrus species,* un extrait de *Paullinia species* et un extrait de *Theobroma species.*

2. Compositions selon la revendication 1, comprenant à titre de principes actifs un mélange consistant en un extrait hydroalcoolique *d'Allium species,* un extrait hydroalcoolique de *Citrus species,* un extrait hydroalcoolique, atomisé ou non, de *Paullinia species* et un extrait hydroalcoolique, atomisé ou non, de *Theobroma species.*

3. Procédé de préparation des compositions telles que définies selon la revendication 1 ou 2, **caractérisé en ce que** l'on prépare une liqueur mère renfermant les extraits *d'Allium species* et de *Citrus species,* puis mélange cette liqueur mère, avec un extrait de *Paullinia species* et un extrait de *Theobroma species.*

4. Procédé de préparation selon la revendication 3, **caractérisé en ce que** la liqueur mère est préparée par épluchage, nettoyage, broyage et macération des *Alliums* et des *Citrus* pendant plusieurs heures dans l'alcool, filtration puis ajustement de l'extrait alcoolique et mélange avec les autres ingrédients.

5. Utilisation cosmétique non-thérapeutique des compositions telles que définies selon la revendication 1 ou 2, pour ralentir la chute des cheveux, stimuler leur croissance et augmenter la densité des cheveux.

6. Compositions selon la revendication 1 ou 2 pour utilisation comme médicament pour ralentir la chute des cheveux, pour stimuler leur croissance et augmenter leur densité.

## Patentansprüche

1. Zusammensetzungen, die als aktive Prinzipien ein Gemisch umfassen, das aus einem Extrakt von *Allium-*Arten, einem Extrakt von *Citrus*-Arten, einem Extrakt von *Paullinia-Arten* und einem Extrakt von *Theobroma-*Arten besteht.

2. Zusammensetzungen nach Anspruch 1, die als aktive Prinzipien ein Gemisch umfassen, das aus einem wässrig-alkoholischen Extrakt von *Allium-Arten,* einem wässrig-alkoholischen Extrakt von *Citrus*-Arten, einem wässrig-alkoholischen, zerstäubten oder nichtzerstäubten Extrakt von *Paullinia-Arten* und einem wässrig-alkoholischen, zerstäubten oder nichtzerstäubten Extrakt von *Theobroma-Arten* besteht.

3. Verfahren zur Herstellung von Zusammensetzungen wie nach Anspruch 1 oder 2 definiert, **dadurch gekennzeichnet, dass** ein Mutterlikör hergestellt wird, der die Extrakte von *Allium*-Arten und von *Citrus*-Arten enthält und der Mutterlikör daraufhin mit einem Extrakt von *Paullinia*-Arten und einem Extrakt von *Theobroma*-Arten gemischt wird.

4. Verfahren zur Herstellung nach Anspruch 3, **dadurch gekennzeichnet, dass** der Mutterlikör hergestellt wird durch Schälen, Reinigen, Zerkleinern und Mazerieren von *Allium* und von *Citrus* während mehrerer Stunden in Alkohol, Filtrieren und daraufhin Anpassen des alkoholischen Extrakts und des Gemischs mit den anderen Inhaltsstoffen.

5. Kosmetische nicht-therapeutische Verwendung von Zusammensetzungen wie nach Anspruch 1 oder 2 definiert, um den Ausfall der Haare zu verzögern, deren Wachstum zu stimulieren und die Haardichte zu erhöhen.

6. Zusammensetzungen nach Anspruch 1 oder 2 zur Verwendung als Arzneimittel zum Verzögern des Haarausfalls, zum Stimulieren deren Wachstums und zum Erhöhen derer Dichte.

## Claims

1. Compositions comprising as active ingredients, a mixture consisting in an extract of *Allium species,* an extract of *Citrus species,* an extract of *Paullinia species* and an extract of *Theobroma species.*

2. Compositions according to Claim 1, comprising as active ingredients a mixture consisting in an aqueous-alcoholic extract of *Allium species,* an aqueous-alcoholic extract of *Citrus species,* an aqueous alcoholic extract, atomised or not, of *Paullinia species* and an aqueous-alcoholic extract, atomised or not, of *Theobroma species.*

3. A method for the preparation of compositions as defined in claim 1 or 2, **characterised by** the preparation of a master solution containing the extracts of *Allium species* and *Citrus species* followed by the mixing of this master solution with an extract of *Paullinia species* and an extract of *Theobroma species.*

4. A method of preparation according to Claim 3, **characterised in that** the master solution is prepared by picking, cleaning, milling and macerating the *Allium* and the *Citrus* for several hours in alcohol, filtering the mixture, adjusting the alcoholic extract and mixing with the other ingredients.

5. The non-therapeutic cosmetic use of compositions as defined in claim 1 or 2 for slowing down the loss of hair, stimulating its growth and increasing the density of hair.

6. Compositions according to claim 1 or 2 for use as medicine for slowing down the loss of hair, stimulating its growth and increasing the density of hair.
